# EUROPÄISCHE PATENTANMELDUNG

(11) **EP 0 587 002 A1**
(43) Veröffentlichungstag der Anmeldung: **16.03.1994**
(21) Anmeldenummer: 93113817.6
(22) Anmeldetag: 30.08.1993
(51) Int. Cl.: A61K 47/10, A61K 9/00

(54) **Thymol und/oder Carvacrol Konservierungsmittel für Injektionsformulierungen**

(30) Priorität: 10.09.1992 DE 4230268
(71) Anmelder: BAYER AG, 51368 Leverkusen (DE)
(72) Erfinder: Wetzstein, Heinz-Georg, Dr., D-51377 Leverkusen (DE)

(57) **Zusammenfassung**

Die vorliegende Erfindung betrifft neue Konservierungsmittel für Injektionsformulierungen insbesondere Vakzine auf Basis Thymol oder Carvacrol.

## Beschreibung

Die vorliegende Erfindung betrifft neue Konservierungsmittel für Injektionsformulierungen auf Basis von Thymol und/oder Carvacrol.

Arzneimittel und insbesondere Injektionsmittel insbesondere in Mehrdosisbehältnissen müssen gegen Mikroorganismen wie Bakterien, Hefen, Pilze geschützt werden.

Die Zugabe von Konservierungsmittel zu Arzneimitteln ist bekannt. So werden Injektionsmittel durch Alkohole, Formaldehyd, Säuren, p-Hydroxybenzoesäure-Ester, Phenole und Kresole sowie organische Quecksilberverbindungen konserviert (Wallhäußer Pharm. Ind. 47 (1985) S. 191-202). Thymol ist zur Konservierung von oral oder dermal verabreichbaren Mitteln bereits verwendet worden (loc. cit.). Es ist jedoch noch nichts über ihre Verwendung zur Konservierung von Injektionsmitteln bekannt geworden obwohl Phenol für diese Zwecke eingesetzt wird. Andererseits findet sich in Mertindale 29. Auflage, Seite 971, unter dem Stichwort Thymol der Hinweis, daß die Verwendbarkeit von Thymol dadurch eingeschränkt ist, daß Thymol nur schwer wasserlöslich ist, gewebereizend ist und seine Wirkung durch Proteine vermindert wird.

Da Injektionsmittel zumeist wäßrig sind, direkt in Gewebe eingebracht werden und oft, wie im Falle von Vakzinen, Proteine als Wirkstoff enthalten, muß ein solcher Hinweis dazu führen, daß Thymol für solche Konservierungszwecke als ungeeignet angesehen wird.

Die in der Praxis eingesetzten Konservierungsmittel in Injektionsmitteln auf Basis Phenol und Kresol sind aufgrund der Toxizität von Phenol und Kresol sowie ihrer eher mikrobistatischen Wirkung unbefriedigend.

Konservierungsmittel auf Basis organischer Quecksilberverbindungen bereiten ebenfalls Schwierigkeiten z.B. da sie im Fall unverbrauchter Mittel sehr aufwendig - und damit kostspielig - entsorgt werden müssen.

Es bestand daher ein dringendes Bedürfnis nach einem leicht zugänglichen, wenig toxischen und ohne besondere Auflagen zu entsorgenden Konservierungstoff für Injektionslösungen, insbesondere für Vakzinen.

Die vorliegende Erfindung betrifft:
1. Neue Konservierungsmittel für Injektionsmittel auf Basis Thymol und/oder Carvacrol.
2. Neue Injektionsmittel die Thymol und/oder Carvacrol als Konservierungsmittel enthalten.
3. Die Verwendung von Thymol und/oder Carvacrol zur Konservierung von Injektionsmitteln.
4. Die Verwendung von Thymol und/oder Carvacrol zur Herstellung von haltbaren Injektionsmitteln.

Injektionsmittel sind parenteral verabreichbare Mittel in der Human- und Veterinärmedizin. Insbesondere gehören dazu Mittel, die Wirkstoff in Wasser gelöst oder suspendiert enthalten oder in öligen Emulsionen enthalten sind und denen vor ihrer Anwendung Wasser zur Verdünnung zugesetzt wird. Besonders genannt seien Vakzinen und Mittel, die in Mehrdosisbehältern aufbewahrt werden.

Injektionsmittel sind aber auch Rekonstitutionsmittel wie z.B. Wasser oder physiologische Kochsalzlösungen, die zur Rekonstitution von Injektionsmitteln dienen.

Als Injektionsmittel seien insbesondere genannt Mittel, die Wirkstoffe enthalten, die im sauren oder basischen Bereich instabil sind und für die Alkohol als Konservierungsmittel nicht in Betracht kommt. Dazu gehören Bakterizide, Fungizide, Betäubungsmittel, Anthelmintika, Roborantien, Blutersatzmittel, Diagnostika, Enzyme, immunoloqisch wirksame Produkte wie Vakzinen z.B. auf Basis von Toxinen, Toxoiden, monoklonalen Antikörpern, Antiseren, Antitoxinen, inaktivierten Virus- oder Bakterienkulturen. Besonders hervorgehoben seien Vakzinen.

Thymol und Carvacrol sind bekannte Substanzen. Besonders bevorzugt wird Thymol. Als Konservierungsmittel in Injektionsmitteln werden Thymol und Carvacrol in Mengen von 0,01 bis 0,1 Gew.-%, bevorzugt von 0,02 bis 0,07 Gew.-%, bezogen auf die Endformulierung, eingesetzt.

Ein typisches erfindungsgemäßes Injektionsmittel hat die Zzusammensetzung:

| | |
|---|---|
| Inaktivierte Virussuspension | 25 bis 95 Gew.-% |
| Adjuvans | 0,1 bis 75Gew.-% |
| Thymol | 0,02 bis 0,05 Gew.-% |
| Hilfsstoffe | 0,000001 bis 0,001 Gew.-% |
| Wasser in Injektionszwecken | ad 100 Gew.-% |

Als Adjuvanzien dienen die bei Vakzinen bekannten Adjuvanzien wie Aluminiumhydroxide, Saponin, Öle oder Bakterienextrakte wie z.B. Freund'sches Adjuvans.

Als Hilfsstoffe seien genannt die für Einsatz in Impfstoffen zugelassenen Farbstoffe sowie Stabilisatoren z.B. Polygeline.

Die Britische Pharmacopöe (1988), Appendix XVI C A 200 bis A 203, fordert für humane Parenteralia in Mehrdosisbehältnissen die folgenden Ergebnisse im Konservierungsbelastungstest um die Konservierungsgüte nachzuweisen.

Für Bakterien: Nach 6 Stunden muß eine Keimzahlreduktion um 3 Zehnerpotenzen erfolgt sein; nach 24 Stunden und danach darf kein Mikroorganismus in 1 ml Präparat mehr nachweisbar sein.

Für Hefen und Pilze: Innerhalb von 7 Tagen muß eine Keimreduktion um mindestens 2 Zehnerpotenzen erreicht werden; danach darf es zu keinem erneuten Anstieg der Keimzahl kommen.

In Versuchen wurde die Konservierungsgüte von erfindungsgemäßen Probeformulierungen im Vergleich zu Formulierungen, die mit Merthiolat konserviert wurden, untersucht.

Dazu wurden 4 Chargen des Paramunitätsinducers Baypamun® flüssig mit unterschiedlichen Thymolkonzentratzonen hergestellt und nach den Forderungen der Britischen Pharmacopöe 1988 untersucht.

Die Charge D1 (0,01 % Thymol) erfüllte diese sehr strengen Forderungen nicht vollständig. In einem Zeitraum vom 7 Tagen kam es bei den Bakterien zu einem geringfügigen Anstieg der Keimzahl. Nach 28 Tagen war die Ausgangsbakterienzahl um 1 bis 3 Zehnerpotenzen reduziert worden. In Bezug auf Hefen und Pilze zeigte sich ein fungistatisches Wirkprinzip. So kam es innerhalb von 28 Tagen weder zum Anstieg der Keimzahlen, noch zu einer bemerkenswerten Reduktion.

Sehr stark mikrobizide Effekte wurden bei der Untersuchung der Charge D4 (0,1 % Thymol) deutlich: Nach 6 Stunden waren Bakterien in 1 ml nicht mehr nachweisbar, ebenso Candida albicans nach 1 Tag und Aspergillus niger nach 7 Tagen. Danach kam es zu keinem erneuten Anstieg der Keimzahl.

Charge D2, mit 0,05 % Thymol konserviert, zeigte ebenfalls eine zufriedenstellende Absterbekinetik, die jedoch weniger drastisch verlief als bei Charge D4. Nach 1 Tag kam es zwar bei Staphylococcus aureus nicht zu einer völligen Keimfreiheit in 1 ml Probe, Pseudomonas aeruginosa und Candida albicans waren jedoch nicht mehr nachweisbar.

Da die auftretenden Keimzahlen jedoch sehr gering waren, wären in diesem Fall die Anforderungen der BP 1988 erfüllt, zumal sich nach 7 Tagen eine Keimfreiheit (Bakterien, Hefen, Pilze) ergab.

Bei Konservierung mit 0,025 % Thymol (Charge D3) zeigte sich ebenfalls eine stark mikrobizide Wirkung. Allerdings wurden auch hier die Forderungen bezüglich der Abtötung der Bakterien nach 1 Tag nicht vollständig erfüllt; nach 6 Stunden hatte jedoch eine Bakterienreduktion um 3 Zehnerpotenzen stattgefunden. Die Absterbekinetik der Hefen und Pilze entsprach den Forderungen der BP 1988.

Zum Vergleich wurde die Konservierungsgüte einer mit 0,01 % Merthiolat konservierten Charge desselben Paramunitätsinducers untersucht. Es zeigte sich hier ein stark unterschiedliches, keimspezifisches Absterbeverhalten. Zufriedenstellende Resultate ergaben sich für Pseudomonas aeruginosa, Candida albicans und Aspergillus niger. In dieser Hinsicht entsprach die Konservierungsgüte den Anforderungen der BP 1988. Bei Staphylococcus aureus und Escherichia coli ergab sich zunächst eine bakteriostatische, nach 7 Tagen jedoch eine mikrobizide Wirkung.

Die beiden untersuchten Chargen wiesen keine signifikanten Unterschiede hinsichtlich der Absterbekinetiken auf. Die Forderungen des Deutschen Arzneibuchs (10. Ausgabe, 1991) sowie des US Pharmacopeia XXII wurden von den Chargen D₂, D₃ und D₄ erfüllt. Die mit 0,01 % Merthiolat Konservierte Charge entsprach lediglich den Anforderungen für Hefen und Schimmelpilze.

Die vorliegende Erfindung soll durch die nachfolgenden Beispeile noch näher beschrieben werden:

### Beispiel 1

17,9 l inaktivierter Suspension des Paramunitätsinducers Parapox ovis werden 0,2 mg Phenolrot, 500 g Polygeline und destilliertes Wasser ad 20 l hinzugefügt. In dieser Suspension wurde 10 g Thymol eingerührt.

### Beispiel 2

| | |
|---|---|
| Inaktivierte IBR-Virussuspension | 60 Gew.-% |
| Aluminiumhydroxidsuspension 3 % | 31 Gew.-% |
| Saponin | 0,125 Gew.-% |
| Thymol DAB | 0,025 Gew.-% |
| Physiologische Kochsalzlösung (phosphatgepuffert) | 8,850 Gew.-% |

### Beispiel 3

In 90 l Aqua p.i. werden nacheinander gelöst: 800 g NaCl p.a., 20 g KCl p.a., 20 g KH₂PO₄ p.a., 290 g Na₂HPO₄ x 12 H₂O p.a. und 50 g Thymol DAB. Der pH wird mit 1 n NaOH bzw. 1 n HCl auf 7,3 eingestellt. Abschließend wird mit Aqua p.i. ad 100 l aufgefüllt.

## Patentansprüche

1. Konservierungsmittel für Injektionsmittel auf Basis Thymol und/oder Carvacrol.

2. Injektionsmittel die Thymol und/oder Carvacrol als Konservierungsmittel enthalten.

3. Verwendung von Thymol und/oder Carvacrol zur Konservierung von Injektionsmitteln.

4. Verwendung von Thymol und/oder Carvacrol zur Herstellung von haltbaren Injektionsmitteln.
